# EUROPEAN PATENT SPECIFICATION

(11) **EP 0 316 771 B1**
(45) Date of publication and mention of the grant of the patent: **02.08.1995**
(21) Application number: 88118757.9
(22) Date of filing: 10.11.1988
(51) Int. Cl.: A61F 13/20

(54) **Pad for absorption of human exudate**
Binde für die Absorption von menschlichem Exsudat
Couche d'absorption d'exsudat humain

(30) Priority: 20.11.1987 US 123483
(43) Date of publication of application: 24.05.1989
(73) Proprietor: KIMBERLY-CLARK CORPORATION, Neenah, Wisconsin 54956-0349 (US)
(72) Inventor: Sherrod, Earle Harry, Appleton Wisconsin 54911 (US); Lassen, Frederick Oma, Neenah Wisconsin 54956 (US)
(74) Representative: Diehl, Hermann O. Th., Dr.

(56) References cited:
- GB-A- 2 087 240
- GB-A- 2 124 907
- US-A- 4 531 945

## Description

The present invention relates to pads for absorption of human exudate, especially to a feminine pad.

In the use of sanitary napkins or pads for absorption of human exudate, there has been a problem in that the napkin is provided with much more absorbent capacity than is normally utilized by the user of the pad. The menstrual fluid tends to be distributed in the longitudinal middle of the pad. Further, the fluid in the middle section of the pad tends to be on the top of the pad rather than in the lower absorbent portion. This concentration of the fluid in the middle portion of the pad leads to premature failure as fluid may leak over the edge of the pad or the pad may leak when it is deformed by movement of the wearer.

Attempts have been made to solve this problem by making sanitary napkins so that they more closely follow the shape of the body and have their greatest absorption capacity where the need is greatest. However, even with such pads side leakage is still a substantial problem. Further, the problem of the absorption capacity of the pad not being used because of concentration of menstrual fluids in the upper or body side of the absorbent rather than down towards the back of the absorbent remains a problem.

Another problem with conventional sanitary pads as well as incontinence pads is that if fluid is applied to the pad at a rapid rate the pad is often unable to absorb the fluid rapidly enough and the fluid will run off the sides of the pad.

It has been proposed in U.S. 4,333,462 - Holtman that a pad having center reservoirs be formed in order to provide for receiving body fluid and its absorption. U.S. 4,029,101 - Chesky et al. discloses a pad having an open longitudinal center slot that allows fluid to enter into the midportion of the pad. It is also disclosed in Chesky et al. that the pad may be provided with an elongated baffle to wick material laterally within the pad.

US-A-4 531 945 discloses a sanitary napkin which is provided with three layers of absorbent material with the first and third layers being substantially indentical and having areas of contact through one opening in the second layer.

There remains a need for a pad that will make more efficient use of the absorbent available in the pad. Further, there remains a need for a pad that will better resist bending and twisting in order to present correct placement for delivery of exudate to the pad. A need also exists for a pad that will resist side leakage if the pad is twisted.

It is, therefore, an object of the invention to overcome disadvantages of prior pads for absorption of human exudate. This object is solved by the pad described in the independent claim 1. Further advantageous features of the pad are evident from the dependent claims.

The invention provides a pad that also reduces bunching and twisting. Further, the invention provides a feminine pad that has reduced side leakage.

Another additional feature of the invention is that it provides a feminine pad having efficient use of absorbents.

The pad of the invention is particularly absorbent for the perineum and in particular feminine care pads with a construction allowing improved distribution of fluids.

The invention, therefore, relates to an absorbent product, such as a sanitary napkin or incontinence product, comprising an absorbent body and a fluid-permeable layer located closest to the user when in use and a fluid-impermeable layer disposed on the opposite side - the back - of the absorbent body.

According to the invention it is provided a pad that is comprised of a permeable cover and an impermeable backing member with an absorbent therebetween. The pad is further provided with a fluid transfer or fluid movement structure that is on at least a part of the surface of the absorbent between the permeable member and the absorbent, extends down through the absorbent and extends to the area between the impermeable backing member and the absorbent. The fluid transfer member is a material that wicks fluids but has at least some hydrophobic component that allows the fluids to be easily transferred from the fluid movement member to a more hydrophilic absorbent such as wood fluff that will store and contain the fluid. The preferred fluid transfer member may be formed of carded webs of rayon or cotton blended with polypropylene or of meltblown polypropylene with pulp. Furthermore, a liquid storage member comprising superabsorbent is located adjacent said impermeable backing member.

Further details and advantages of the invention will become evident from the enclosed drawings and their description:
Figure 1 is a top view of a feminine pad.
Figure 2 is a side view of a pad.
Figure 3 is an end view of a pad.
Figure 4 is a cross-sectional view of the pad of Figure 1 on cross-sectional line 4-4.
Figure 5 is a cross-sectional view of the pad on cross-sectional line 5-5 of Figure 1.
Figure 6 is a cross-sectional view of an alternate pad design.
Figure 7 is a cross-sectional view of an alternate pad design. The embodiments of Figures 4 to 7 do not comprise the features of the present invention but are usefull for understanding it.
Figure 8 is a top view of a prior art pad illustrating the stain area.
Figure 9 is a cross-sectional view of the pad of Figure 8 on cross-sectional line 9-9.
Figure 10 is a cross-sectional view of an embodiment of a pad in accordance with the invention.
Figure 11 is a cross-sectional view of a pad in accordance with the invention.

The pads of the instant invention have numerous advantages over prior pads for absorption of human exudate, particularly those used as catamenial devices. The pads of the instant invention provide transport of menstrual fluids down through the pads such that the fluids are entrapped within the pad itself and do not reside near the surface where they may be squeezed out by movement of the wearer. The pads of the invention also provide a cleaner appearance as the stain does not spread as far on the surface as it typically would with prior pads. The pads of the invention also are less likely to leak from the sides, particularly prior to a great deal of fluid being absorbed by the pad. Fluids are transported to the center and underside of the pad rather than remaining at or near the surface of the pad where they may cause leakage. These and other advantages, such as an increased rate of absorbency, will be apparent from the description of the drawings and explanation below.

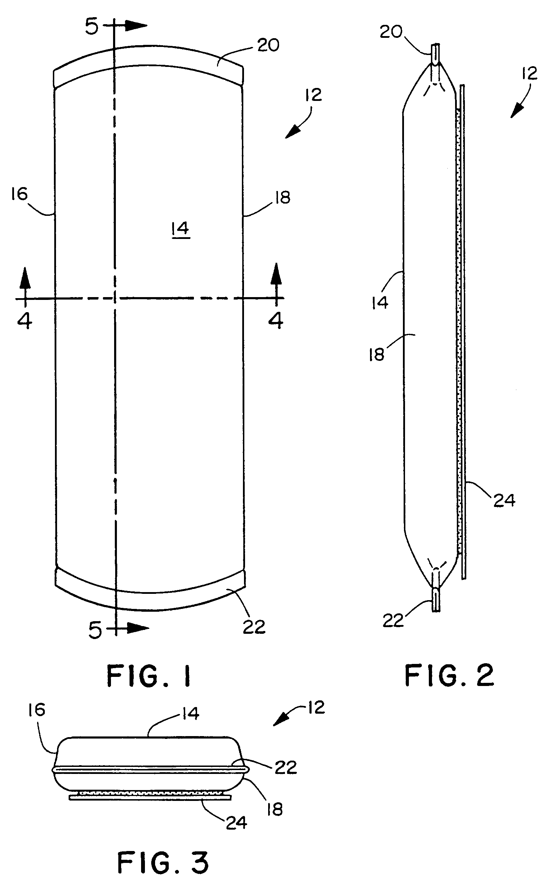

Figure 1 is a top view of a feminine pad 12. The pad has a bodyside cover 14 and longitudinal edges 16 and 18. The pad is gathered at ends 20 and 22. As shown in Figures 2 and 3 the pad 12 is provided with a peel strip 24. The peel strip 24 is removed prior to placement of the pad in the user's undergarment.

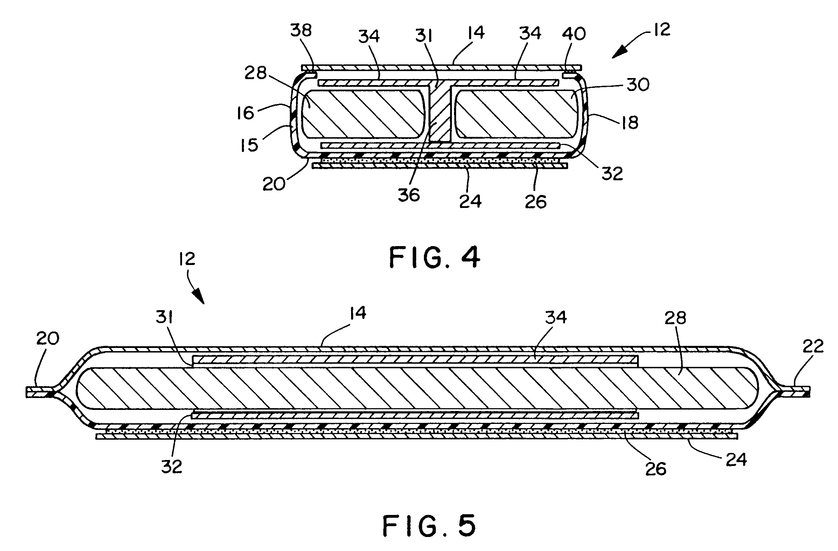

Figs. 4 to 7 illustrate several arrangements of the transfer material. In these figures the liquid storage member 180 and 212 is not shown.

Figure 4 is a cross-sectional view on line 4-4 of pad 12 in Figure 1. Pad 12 is provided with cover 14 and an impermeable member 15 that forms sides 16 and 18 and backing 20. The peel strip 24 covers garment attachment adhesive 26. The inner portion of the napkin 12 is formed of absorbents 28 and 30. The fluid movement structures 31 and 32 are formed of the same or similar materials. When considered together absorbents 31 and 32 form an I-beam shape cross-section. For best performance the absorbents 31 and 32 should be in contact to provide fluid transfer. The material forming structures 31 and 32 will rapidly absorb fluid and transfer it throughout pad 12. However, the fluid movement structures 30 and 32 will give up fluid easily to the absorbents 28 and 30. Structure 31 is composed of a surface portion 34 that overlies the absorbents 28 and 30. It is further provided with a centered member 36 between absorbents 28 and 30 providing fluid movement from the top of the bottom of pad 12. Member 31 will gather fluids at the surface of the pad and transfer them down through the central portion of the pad to the bottom fluid movement member 32 as well as into the reservoir members 28 and 30. The cover member 14 is adhesively connected to an impermeable member 15 in areas 38 and 40. Alternatively the pad could be wrapped by the permeable member and sealed at the bottom. As illustrated in the Figure 5 cross-sectional view of pad 12 on line 5-5 of Figure 1, the fluid movement members 31 and 32 do not extend the entire longitudinal length of the pad, but are located in the center one-half to two-thirds of longitudinal length.

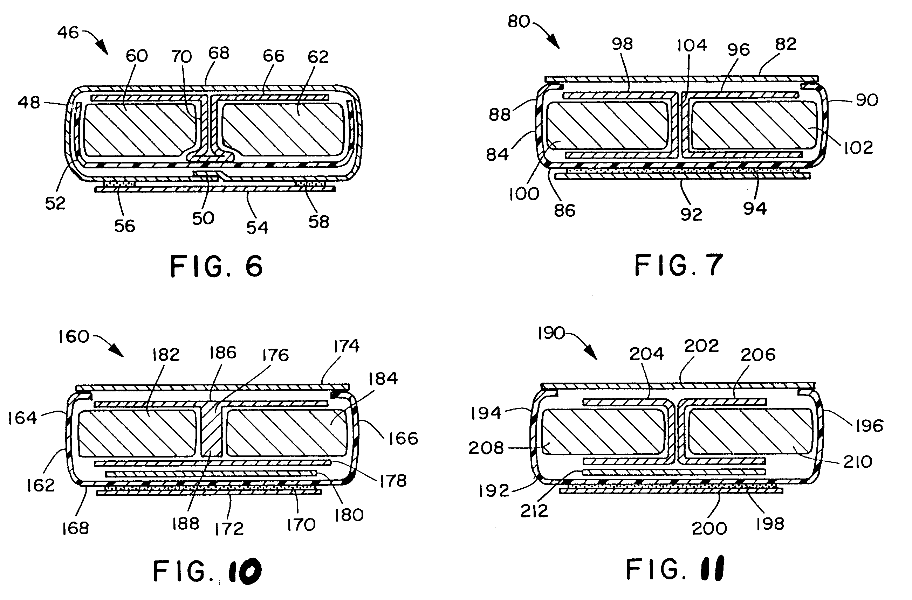

Figure 6 is a cross-sectional view of an alternate embodiment pad. In the embodiment of Figure 6 the pad 46 is provided with a permeable cover material 48 that is wrapped around the entire pad and overlapped with adhesive connection at 50. The pad has an impermeable baffle 52 and a peel strip 54. The peel strip covers two parallel lines of adhesive 56 and 58 for garment attachment. Alternatively a single wide band of adhesive could be used rather than multiple lines as shown. The absorbent comprises two reservoir absorbent members 60 and 62. The fluid transfer or fluid movement member 66 extends between the bodyside surface 68 and absorbent 60 and 62. The fluid transfer member 66 further is provided with a folded portion 70 extending down between the absorbent members 60 and 62 for distributing fluids down into the pad and into the lower portion of the reservoir absorbents 60 and 62.

Figure 7 illustrates in cross-section another alternate pad 80. Pad 80 has a permeable bodyside member 82 and an impermeable member 84 forming the backside 86 and the two longitudinal sides 88 and 90. The pad is provided with peel strip 92 covering the garment attachment adhesive strip 94. The absorbents comprise fluid movement members 96 and 98 as well as the fluid holding or reservoir absorbents 100 and 102. Fluid movement member 98 is wrapped around the portion of absorbent 100 that is towards the center of the pad. Absorbent 96 is wrapped around the portion of absorbent 102 that is towards the center of the pad. Members 96 and 98 provide transfer of fluid down through the center 104 of the pad to the area between the bottom 86 and the absorbents 100 and 102.

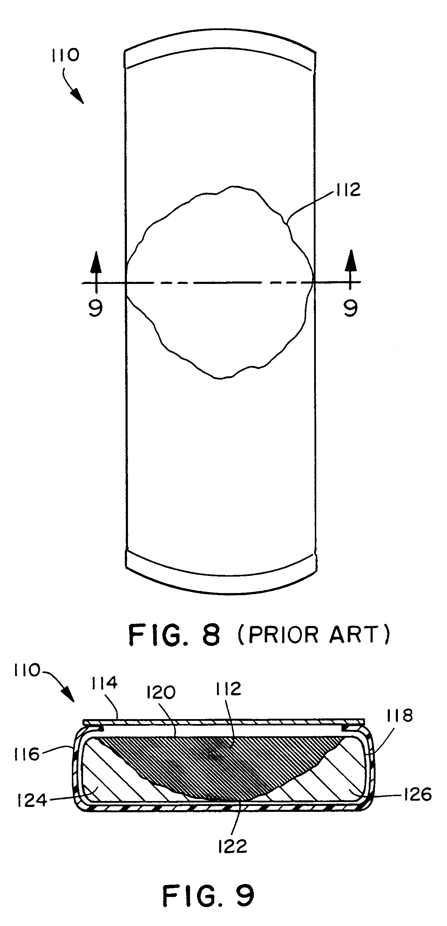

Illustrated in Figures 8 and 9 is a used pad of the prior art. Figure 8 is a top view of a pad 110 of the prior art. Area 112 is a catamenial fluid stain. Figure 9 is a cross-section view on cross-section line 9-9 of pad 110 of Figure 8. As illustrated in cross-section the pad 110 is formed with a bodyside cover 114 and an impermeable baffle 116. The pad further is provided with an absorbent 118 such as wood fluff. The stain 112 is wider at the top 120 than at the bottom portion 122. It generally has the shape of an inverted truncated pyramid. Therefore the absorbent in areas 124 and 126 is wasted and a large stain 112 is on the top of the pad.

Figures 10 and 11 illustrate embodiments of the invention in which absorbent structures such as a superabsorbent layer is utilized. Pad 160 as illustrated in cross-section in Figure 10 is comprised of an impermeable baffle member 162 that forms the sides 164 and 166 and the bottom 168 of the pad 160. The pad in conventional manner is provided with garment attachment adhesive 170 covered by peel strip 172. The bodyside permeable member 174 is placed on the bodyside of the pad. The absorbents comprise fluid movement members 176 and 178. Liquid storage layer 180 comprises a superabsorbent containing sheet. Absorbents 182 and 184 are a bulk absorbent such as wood fluff. The I-beam shape fluid movement members 176 and 178 provide movement of the fluid from the top surface of the member 186 through the middle portion of the fluid movement member 188 to the bottom 178. New layer 180 may be an absorbent system such as a sheet of superabsorbent, a microfiber sheet or may be a carrier for superabsorbent particles. Superabsorbents generally are hydrocolloidal polymers that have the ability to absorb greater than thirty times their weight in fluid. However, the absorption of such fluid is not extremely rapid. Therefore, their placement at the bottom allows time for absorption of fluids from the movement member 178. Illustrated in Figure 11 is a pad 190 that is composed of an impermeable baffle member 192 that forms the sides 194 and 196 of the pad. The pad in conventional manner is provided with a garment attachment adhesive 198 and a peel strip 200. The bodyside surface is provided with a permeable member 202. Fluids as they pass through permeable member 202 contact the fluid movement members 204 and 206 that are wrapped around the inner portions of the absorbents 208 and 210. A superabsorbent containing layer 212 is provided beneath the lower portion of the fluid transfer members 204 and 206.

The fluid movement structure or fluid transfer material may be any material that will readily transfer fluids as well as give up fluids to absorbents such as wood fluff. These materials generally have a hydrophilic and a hydrophobic component. Suitable materials are bonded carded webs, coform and meltblown materials. Coform is an air formed blend of meltblown polymer and staple fibers, most commonly meltblown polypropylene and devillicated wood fibers.

Preferred materials include ultrasonically bonded carded webs that are blends of polypropylene, polyester and rayon. Another preferred material is macrofiber coform which may be defined as coform that has a fiber diameter of greater than about 15»m diameter. Another preferred material is a bonded carded web of a blend of polyester and rayon. The transfer material should have a hydrophilic component to facilitate the flow of fluids within the transfer layer. However it should also include a hydrophobic component in order that the fluids will preferentially transfer to other absorbents that are more uniformly hydrophilic rather than be retained. Further, it is possible that some of all of the fibers forming the transfer members may be treated prior or during formation of the carded web or coform in order to make them hydrophilic or hydrophobic as the case may be. It is possible that the coform materials may be blends of staple fibers such as rayon as well as wood in combination with the air-formed polypropylene fibers. A bonded carded web of rayon and polyester blend that has been spot bonded to a substrate of spunbond polypropylene also may be utilized for transfer.

The material selected as the reservoir or bulk absorbent that absorbs the fluids from the transfer members in these products may be any material that will preferentially absorb fluids from the transfer layers. Further such materials should not preferentially cause flow of liquid from the reservoir absorbent back to the transfer material and therefore may be any absorbent with good liquid holding ability and the ability to preferentially absorb fluids from the transfer member. Typical of such materials are cellulose sponge, polymer sponge, tissue and gauze. The preferred material is wood fluff as it has high liquid holding ability and low cost. It is also possible that superabsorbents or other absorbent materials such as microfibers may be added to the bulk absorbent in order to better hold fluid. Superabsorbents are hydrogel materials that have the ability to hold greater than thirty times their weight of fluid.

The invention has the advantage that the Z-direction transfer is improved such that fluids are quickly and efficiently moved from the surface of the pad between the cover and the bulk absorbent down through the middle of the pad and to the bottom below the bulk absorbent. The bulk absorbent then is able to preferentially absorb fluids from the transfer member. The pads further have the advantage that by having the open area in the middle between the bulk absorbents the pad tends to flex in the middle and is better able to conform to the body, thereby bunching and twisting less. As it flexes with the middle portion lifting upward for better contact, the liquid transfer member then is better exposed to initial contact of the fluid with the pad. The pads of the invention generally do not require a tissue wrapping member around the fluff absorbents. The use of the carded web or meltblown liquid movement members provide sufficient strength that the tissue wrap of the fluff is not necessary. This is a cost savings in that the wrap is not necessary.

Although the invention has been described with specific examples, it will be understood that the description thereof is intended to be illustrative rather than exhaustive. Details of construction may be modified or changed without departing from the spirit or scope of the invention. For instance, the bulk or reservoir absorbent could be formed of layers of different density in order to further segregate absorptive abilities. Further, other layers such as reinforcing layers could be utilized. The invention while described with reference primarily to feminine pads also would be suitable for use in incontinence products or diapers. The invention also would find use in elasticized incontinent garments, diapers or feminine care products that have the longitudinal center edges elasticized to shape the garment.

## Claims

1. A feminine pad comprising a bodyside permeable cover member (14,68,92,174,202), an impermeable backing (15,52,84,162,192) and a bulk absorbent formed from absorbents (28,30,60,62,100,102,182,184,208,210) there-between wherein a transfer material (31,32,36,66,96, 98,176,178,204,206) extends over a portion of said absorbents below said cover, through the longitudinal middle portion of said pad between said absorbents and between said absorbents and said impermeable backing (15, 52,84,162,192)
characterized in
further comprising a liquid storage member (180,212) comprising superabsorbent adjacent said impermeable backing member (162,192) and contacting said transfer material (176,178,204,206) for fluid transfer from said transfer member to said storage member (180,212).

2. The pad of claim 1 wherein said transfer material (176,178,204,206) has an I- beam cross sectional shape.

3. The pad of any one of the preceding claims, wherein said transfer material (176,178,204,206) is a pair of U-shaped members.

4. The pad of any one of the preceding claims wherein said transfer material is selected from the group consisting of carded webs of polyester fibers, rayon fibers, polypropylene fibers and mixtures thereof.

5. The pad of any one of the preceding claims, wherein said transfer material (176,178,204,206) is a blend of hydrophobic and hydrophylic fibers.

6. The pad of any one of the preceding claims, wherein said transfer material (176,178,204,206) is an air-formed blend of meltblown polymer and staple fibers.

7. The pad of any one of the preceding claims wherein said absorbents (28,30) are located either side of a portion (36) of said transfer material (176,178,204,206) extending down the longitudinal center of said pad (12).

8. The pad of any one of the preceding claims wherein said transfer member (176,178,204,206) extends between about one-half and about two-thirds the length of said pad (12).

## Patentansprüche

1. Monatsbinde, umfassend ein körperseitiges Abdeckelement (14, 68, 92, 174, 202), eine undurchlässige Rückschicht (15, 52, 84, 162, 192) und dazwischen einen aus Saugkörpern (28, 30, 60, 62, 100, 102, 182, 184, 208, 210) geformten Volumensaugkörper, wobei sich ein Übertragungsmaterial (31, 32, 36, 66, 96, 98, 176, 178, 204, 206) über einen Teil der Saugkörper unter der Abdeckung, durch den Mittellängsteil der Binde zwischen den Saugkörpern und zwischen den Saugkörpern und der undurchlässigen Rückschicht (15, 52, 84, 162, 192) erstreckt,
dadurch gekennzeichnet,
daß sie des weiteren ein Flüssigkeitsspeicherelement (180, 212) umfaßt, das ein in der Nähe der undurchlässigen Rückschicht (162, 192) liegendes superabsorbierendes Material umfaßt und das Übertragungsmaterial (176, 178, 204, 206) zur Übertragung der Flüssigkeit vom Übertragungselement in das Speicherelement (180, 212) berührt.

2. Monatsbinde nach Anspruch 1, wobei das Übertragungsmaterial (176, 178, 204, 206) einen I-trägerförmigen Querschnitt hat.

3. Monatsbinde nach einem der vorhergehenden Ansprüche, wobei das Übertragungsmaterial (176, 178, 204, 206) aus einem Paar U-förmiger Elemente besteht.

4. Monatsbinde nach einem der vorhergehenden Ansprüche, wobei das Übertragungsmaterial aus der Gruppe bestehend aus kardierten Vliesen aus Polyesterfasern, Reyonfasern, Polypropylenfasern und Mischungen daraus ausgewählt ist.

5. Monatsbinde nach einem der vorhergehenden Ansprüche, wobei das Übertragungsmaterial (176, 178, 204, 206) eine Mischung aus hydrophilen und hydrophoben Fasern ist.

6. Monatsbinde nach einem der vorhergehenden Ansprüche, wobei das Übertragungsmaterial (176, 178, 204, 206) eine luftgeformte Mischung aus schmelzgeblasenem Polymer und Stapelfasern ist.

7. Monatsbinde nach einem der vorhergehenden Ansprüche, wobei die Saugkörper (28, 30) auf beiden Seiten eines Teiles (36) des Übertragungsmaterials (176, 178, 204, 206), wobei der Teil sich im longitudinalen Zentrum der Binde (12) nach unten erstreckt, angeordnet sind.

8. Monatsbinde nach einem der vorhergehenden Ansprüche, wobei das Übertragungselement (176, 178, 204, 206) sich zwischen ungefähr der Hälfte und ungefähr zwei Drittel der Länge der Binde (12) erstreckt.

## Revendications

1. Masse absorbante d'hygiène intime féminine comprenant un élément d'enveloppe perméable (14, 68, 92, 174, 202) disposé du côté corporel, un support imperméable (15, 52, 84, 162, 192) et un corps absorbant constitué d'absorbants (28, 30, 60, 62, 100, 102, 182, 184, 208, 210) disposés entre ceux-ci, dans laquelle un matériau de transfert (31, 32, 36, 66, 96, 98, 176, 178, 204, 206) s'étend sur une portion desdits absorbants au-dessous de ladite enveloppe, à travers la portion centrale longitudinale de ladite masse absorbante, entre lesdits absorbants, et entre lesdits absorbants et ledit support imperméable (15, 52, 84, 162, 192)
caractérisé en ce qu'elle comprend en outre un élément de stockage des liquides (180, 212) formé d'un superabsorbant adjacent audit support imperméable (162, 192) et en contact avec ledit matériau de transfert (176, 178, 204, 206) pour transférer un fluide depuis ledit élément de transfert vers ledit élément de stockage (180, 212).

2. Masse absorbante selon la revendication 1, dans laquelle ledit matériau de transfert (176, 178, 204, 206) a une section transversale en forme de I.

3. Masse absorbante selon l'une quelconque des revendications précédentes, dans laquelle ledit matériau de transfert (176, 178, 204, 206) est une paire d'éléments en forme de U.

4. Masse absorbante selon l'une quelconque des revendications précédentes, dans laquelle ledit matériau de transfert est choisi dans le groupe consistant en les nappes cardées de fibres de polyester, de fibres de rayonne, de fibres de polypropylène et leurs mélanges.

5. Masse absorbante selon l'une quelconque des revendications précédentes, dans laquelle ledit matériau de transfert (176, 178, 204, 206) est un mélange de fibres hydrophobes et hydrophiles.

6. Masse absorbante selon l'une quelconque des revendications précédentes, dans laquelle ledit matériau de transfert (176, 178, 204, 206) est un mélange formé à l'air de fibres polymères obtenues par fusion-soufflage et de fibres courtes.

7. Masse absorbante selon l'une quelconque des revendications précédentes, dans laquelle lesdits absorbants (28, 30) sont situés de chaque côté d'une portion (36) dudit matériau de transfert (176, 178, 204, 206) qui s'étend de haut en bas de l'axe médian longitudinal de ladite masse absorbante (12).

8. Masse absorbante selon l'une quelconque des revendications précédentes, dans laquelle ledit élément de transfert (176, 178, 204, 206) s'étend sur environ un demi à environ deux tiers de la longueur de ladite masse absorbante (12).
